Europäisches Patentamt

European Patent Office

Office européen des brevets

(11) Publication number: **0 393 035 B1**

(12) **EUROPEAN PATENT SPECIFICATION**

(45) Date of publication of patent specification: **21.06.95**  (51) Int. Cl.[6]: **C12N 9/00**, C12N 9/14

(21) Application number: **88906857.3**

(22) Date of filing: **08.07.88**

(86) International application number:
**PCT/SE88/00374**

(87) International publication number:
**WO 89/01031 (09.02.89 89/04)**

(54) **METHOD FOR THE ISOLATION OF ACTIVE ENZYME(S) FROM KRILL TISSUE.**

(30) Priority: **06.08.87 SE 8703064**

(43) Date of publication of application:
**24.10.90 Bulletin  90/43**

(45) Publication of the grant of the patent:
**21.06.95 Bulletin  95/25**

(84) Designated Contracting States:
**AT BE CH DE FR GB IT LI LU NL SE**

(56) References cited:
**WO-A-84/01715**
**FR-A- 1 015 566**
**GB-A- 377 128**
**GB-A- 1 561 613**
**SE-C- 140 568**

(73) Proprietor: **Aktiebolaget L.H. Forskning**
**Bronsgjutaregatan 13**
**S-421 63 Västra Frölunda (SE)**

Proprietor: **Bioscan AS**
**Teglbrennerv 8**
**N-7013 Trondheim (NO)**

Proprietor: **Parti-Transaction AB**
**Sibyllegatan 18**
**S-114 42 Stockholm (SE)**

(72) Inventor: **MOHR, Viggo**
**S:t Jörgensveita 6A**
**N-7000 Trondheim (NO)**
Inventor: **VINCENT, Jan**
**Ulvsundavägen 49**
**S-161 35 Bromma (SE)**
Inventor: **HELLGREN, Lars**
**Bronsgjutaregatan 13**
**S-421 63 V:A Frölunda (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

Journal of Food Biochemistry, vol.2, issued 1978 (Westport, Connecticut), C-S Chen et al, "Purification and properties of trypsin-like enzymes and a carbosypeptidase A from Euphasia superba", s. pp. 349-366, esp. p. 349 (Abstract) and p. 351 (Extraction of proteases)

A. Gildberg, "Autolysis of fish tissue-general aspects", Thesis, published June 1982, by Institute of Fisheries, University of Tromsö (Norway), s. pp. 75-90, esp. p. 89-90

Rote Liste 1977/78, publ. 1978, by Bundesver-band der Pharmazeuti- schen Industrie e.v., Editio Cantor (Aulendorf(Württ), s. Prepara-tions No 59249 Cb (Pankreon R), 59256 Cb (Festal R) and 59260 Cb (Pankreon R com-positum).

Chemical Abstracts, vol. 89 (1978), abstract no. 55 330a, Biologia (Bratislava) 1978, 33 (6), 485-95 (Eng)

Chemical Abstracts, vol. 90 (1979), abstract no. 35434e, J Chin Biochem Soc 1978, 7 (2), 78-87 (Eng)

Chemical Abstracts, vol. 92, (1980), abstract no. 116420r, Jpn KokaiKoho 79 119 017

Chemical Abstracts, vol. 92 (1980), abstract no. 116421s, Jpn Kokai Tokkyo Koho 79 119 011

Chemical Abstracts, vol. 98 (1983), abstract no. 13723m, Pol PL 115 567

Patent Abstracts of Japan, abstract of JP 58-162524 (A), published 1983-09-27

Comp. Biochem. Physiol. vol. 82B, no. 4, pp. 599-606, 1985, (KNUT KR. OSNES AND VIGGO MOHR), "Peptide hydrolases of antarctic krill, euphausia superba", s. p. 601, right column and table 3, p. 602, left column lines 1-6

Comp. Biochem. Physiol. vol. 83B, no. 4, pp. 801-805, 1986, (KNUT KR. OSNES), "Hydrolases of proteins by peptide hydrolases of antarctic krill, euphausia superba"

Inventor: **KARLSTAM, Björn**
**Spiltvägen 7**
**S-740 30 Björklinge (SE)**
Inventor: **BERGLINDH, Thomas**
**Ripvägen 5**
**S-752 52 Uppsala (SE)**

(74) Representative: **Näsman, Rune B G et al**
**Allied Attorneys Chemical AB**
**Box 27097**
**S-102 51 Stockholm (SE)**

## Description

### Field of invention

This invention relates to an improvement in the isolation of active enzymes from aquatic animals of the order Euphausiaceae, commonly called krill. The method of the invention is adapted for enzymes and enzyme precursors from different krill tissues, particularly for those enzymes that originate from the digestive apparatus. The enzymes to be isolated may be different hydrolases, such as proteases, lipases, nucleases, polysaccharidases etc, and other enzymes that effect breakdown of biologic substances e.g. protein, lipid, polysaccharides and nucleic acids, or their constituents.

### General background

Animals of the order Euphausiaceae, and in particular Antarctic krill represented by Euphausia superba and Euphausia crystallorophias, and North Atlantic krill represented by Meganyctiphanes norvegica and Thyssanoessa species, have become increasingly promising as a source of biologically active substances. They are known to contain very effective hydrolases. The high efficiency of mixtures of endo- and exopeptidases of krill in degrading proteinaceous substrates has been demonstrated (Ellingsen, 1982; Saether, 1986; Ellingsen & Mohr, 1987; and Saether, Ellingsen & Mohr, 1987). The peptide hydrolases of krill effect an extensive breakdown of krill tissues and proteins post mortem, resulting in the release of free amino acids and shorter peptides. Visually this is observed as an autolysis. The phenomenon has been suggested to be employed for the preparation of free amino acids on an industrial scale (Ellingsen & Mohr, 1979).

The conditions effecting autolysis of krill have been extensively studied (Ellingsen & Mohr, 1979; Ellingsen, 1982; Ellingsen & Mohr, 1987 and Saether, Ellingsen & Mohr, 1987). The latter publication relates to North Atlantic krill, whereas the former publications deal with Antarctic krill. The rate of autolysis, as measured by the amount of free amino acids released into solution, depends on a series of factors, e.g. temperature, time of incubation, pH and whether whole krill or homogenate is being autolyzed (Ellingsen, 1982).

Substantial evidence is now starting to accumulate that peptide hydrolases from Antarctic krill are superior as debriding agents for wound compared to the enzyme preparations currently in clinical use for this purpose. The general picture which emerges from studies in vitro and clinically (Hellgren, Mohr & Vincent, 1986 and 1987, respectively) is that the debridement with the krill enzymes proceeds at a higher rate, and results in a more complete breakdown of the necrotic tissue than that obtained by other clinically used enzymatic debriders.

The peptide hydrolases of krill which are of particular interest therapeutically have recently been isolated and studied in considerable detail (Osnes & Mohr, 1985a ; Osnes & Mohr, 1985b; Osnes & Mohr, 1986; Osnes, Ellingsen & Mohr, 1986). As shown in these studies, the major krill peptide hydrolases include three different trypsin-like serine proteinases, two carboxypeptidase A-type of enzymes, two carboxypeptidase B-type of enzymes and one aminopeptidase. The enzymes seem to originate almost exclusively from the digestive tract of the krill, and thus seem to constitute enzymes of the digestive apparatus of the animals (Ellingsen, 1982; Grundseth & V.Mohr, unpublished).

Among other enzyme activities that have been measured in different preparations of krill are various polysaccharidase activities (Karlstam, 1988 and Chen & Gau 1981), lipase activity (Nagayama 1979), ribonuclease activity (Van 1982) etc.

The krill peptide hydrolases seem to possess a property which is highly valuable and essential for the use of the mixture of these enzymes for practical applications. The simple digestive system in krill probably implies that the individual enzymes are mutually protected against the degrading effect of each other. Thus, in contrast to enzymes from the mammalian digestive tract, it has been demonstrated conclusively that the krill peptide hydrolases show considerable inertness to breakdown and loss of activity when they are mixed (Osnes & Mohr, 1985ab; Osnes, 1986; Osnes, Ellingsen & Mohr, 1986).

The krill enzymes which are of particular interest for medical and technical applications are usually water soluble, and can be isolated by extracting whole krill, homogenized krill or parts of krill with either water, or buffered, aqueous solutions, followed by isolation and purification of the individual enzymes or groups of enzymes by suitable, established methods (Osnes & Mohr, 1985a). Although such procedures may be satisfactory for laboratory work, large scale industrial processes based on this procedure may represent a problem. The problems relate to the fact that important species of krill usually have a high lipid content, of which glycerophospholipids may make up a considerable proportion (Ellingsen, 1982; Saether, 1986). When extracting different forms of krill with aqueous solvents glycerophospholipids tend to associate with protein, and after centrifugation such extracts may typically consist of a top layer containing oil, below which is a layer rich in glycerophospholipids and protein, below which is an aqueous phase and, finally, at the bottom, an insoluble sediment.

Due to the protein-glycerophospholipid association the phase separation will be far from distinct. Efficient separation of the aqueous phase containing the enzymes therefor represents a problem in an industrial process. Furthermore, in addition to the enzymes, the aqueous phase contains large amounts of soluble proteins, including muscle proteins of the krill. The separation of active enzymes from other proteins require expensive processing technology and, in addition, the non-enzymatic proteins obtained as by-products in this type of process occur in a form which generally has a low market value. Thus, large-scale isolation procedures based on extraction of fresh krill with water may not secure that the therapeutically and technically important enzymes can be isolated in a way which is economically feasible. These problems may be partly overcome by defatting the unhomogenized krill and/or the homogenized with a lipophilic/hydrophobic solvent (e.g. carbon tetrachloride). However, this way of processing krill will give at least one or two extra steps.

The promising prospects of using the digestive enzymes of krill as novel preparations for medical and technical use, stress the need for effective methods aimed at isolating and purifying the krill enzymes.

The invention

The present invention proposes a novel procedure for isolating active enzymes from krill without facing the drawbacks mentioned above. The invention utilizes the well-documented fact that the digestive enzymes of krill effect an extensive breakdown of the krill tissues post mortem, yielding a liquefied system, comprising an oil, an aqueous and an insoluble phase (= sediment). The invention takes advantage of the fact that the system formed after autolysis under efficient conditions may form distinct phases (phase boundaries). A physical separation can effectively be performed by simple process technology, e.g. centrifugation, and without the problems in particular caused by the protein/glycerophospholipid layer described when extracting fresh krill as outlined previously. Prior art methods have aimed at avoiding autolysis. The invention employs autolysis as a prestep to facilitate separation and extraction. The need for separate defatting steps is minimized.

The method of the invention accordingly is characterized by whole krill, homogenized krill, squeezed krill or similarly treated parts of krill (preferably containing the digestive tract) being permitted to autolyse so that a system comprising an oil phase and an aqueous phase is formed having a distinct phase boundary therebetween. High levels of enzyme activity is retained in the aqueous phase after autolysis. The efficient phase separation is probably due to the degradation of glycerophospholipid by krill phopholipase. After the autolysis step, the enzyme-containing aqueous phase is separated from other phases present, followed by the isolation of the enzyme(s) contemplated by conventional procedures. In case the enzyme(s) to be isolated is partitioned to the oil phase conventional isolation procedures known per se is applied to the oil phase. The separation of the individual phases of the autolysate can be carried out be several different methods. Particularly well suited are those exposing the autolysate to centrifugal forces, but other procedures e.g. sedimentation and flotation may also be applicable.

The yield of enzymes obtained depends on time of incubation (autolysis), temperature, pH, type of krill preparation (whole, homogenized or squeezed krill) and the specific enzyme(s) to be isolated.

Generally the conditions for the autolysis to proceed properly should be as below.

Temperature: The lower limit is 15 °C with a preference for temperatures above 20 °C. The upper limit is 70 °C, perferably below 45 °C. Certain krill enzymes have been shown to be heat-sensitive so that when such enzymes are to be retained in the end product, the temperature has to be carefully selected. For instance, if krill hyaluronidase or krill amylase is to be isolated it is recommended to run the autolysis below 45 °C. The krill proteases are quite heat-stable with a temperature optimum around 55-60 °C. This means that if krill proteases are the important enzymes in the end product, the autolysis can be performed up to 70 °C. If a mixture of enzyme(s) are to be isolated and one of them is heat-sensitive, the temperature should be considerable lower, e.g. below 45 °C. In conclusion the temperature should be selected in the range 15-70 °C, preferably 20-45 °C.

pH: This value should be selected in the range of 6-8,5, although autolysis may also be performed down to pH = 5. The preferred range is 6-7,5. We have performed experiments at the pH-optimum for the proteases (pH = 8,2), in order to work effectively. However, at this pH-value the phase separation after autolysis was not satisfactory. This might indicate that enzymes other than the proteases are important to obtain an efficient autolysis (e.g. phospholipases).

4

Time for incubation: This variable should be selected so as to result in the most economic feasible process. By selecting pH and temperature within the ranges given above incubation times of 1 h - 2 weeks, with preference for 5-48 hours, can be accomplished.

It is important to investigate in pretrial experiments that the combination of temperature, pH and time of incubation will not lead to significant degradation and/or inactivation of the enzyme(s) intended to be isolated. Accordingly each enzyme or enzyme mixture has its own optimal conditions within the above-mentioned limits in order to reach the best quality and yield.

Depending on the particular enzyme or group of enzymes to be isolated, and the purity required, methods well known to the specialist can be used to isolate samples of active enzymes from the autolysate, or from appropriate, individual fractions (phases) concentration and/or separation according to molecular size and shape, electrical charge, functional groups, solubility characteristics, or on a combination of these principles, e.g. membrane technology such as ultrafiltration, gel chromatography, ion exchange chromatography, affinity chromatography, electrophoresis, electrodialysis, precipitation by salts or acids, or selective extraction. Final concentration and removal of solvents from the preparations may be achieved by appropriate methods which do not affect enzyme activity adversely, e.f., membrane technology and freeze drying, respectively.

For the purification of specific enzymes see for instance (peptide hydrolases Osnes & Mohr 1985a, 1985b, 1986, Osnes, Ellingsen & Mohr 1986, Chen et al 1978, and Hellgren, Mohr & Vincent 1985; hyaluronidase, endo-(1,3)-beta-D-glucanase and beta-glucuronidase Karlstam 1987; ribonuclease Van 1982). Our study in the krill field has revealed that the trypsin-like krill proteases can be affinity purified on benzamidine adsorbents and most probably also on adsorbents to which trypsin inhibitors are bound, the krill carboxypeptidases on arginine or phenylalanine (hydrophobic) adsorbents and some polysaccharidases on ConA adsorbents (krill hyaluronidase, beta-glucuronidase and endo-(1,3)-beta-D-glucanase).

Example 1

25 g of frozen, Antarctic krill (Euphausia superba) were thawed at room temperature, and homogenized together with 25 ml of deionised water for 45 sec at room temperature using a Janke & Kunkel Homogenizer TP 18. The homogenate was highly viscous and contained a considerable proportion of particulate material. An aliquot of the homogenate was removed for determination of enzyme activity. The homogenate was incubated at 50 °C for 20 h at the natural pH (about 7) of the homogenate.

After incubation the homogenate was centrifuged at 13 000 g for 40 min in the cold. The centrifuged homogenate consisted of three distinct, and well-separated phases: a top layer of oil red in colour due to carotenoids, a clear, aqueous middle layer, and a particulate bottom layer. The aqueous, middle layer was removed with a pipette in the form of a clear solution with low viscosity. An aliquot of the aqueous phase was taken for determination of enzyme activity.

The proteolytic activity of the homogenized krill and of the aqueous phase of the autolysate, was determined with TAME as a substrate according to the method of Rick, 1974 (Methods of Enzymatic Analysis (H.Bergmeyer ed.)2nd.edn., Vol2,pp. 1013-1023. Academic Press, New York). In accordance with the claims of the invention, the aqueous fraction after autolysis at 50 °C for 20 h contained an enzymic activity corresponding to 95 % of that of the original homogenate prior to autolysis.

Example 2

10 ml of the aqueous phase of the krill autolysate prepared according to Example 1 were subjected to ultrafiltration in order to separate the enzyme preparation from low-molecular weight substances. The separation was carried out in an Amicon ultrafiltration unit, using an Amicon Diaflo Ultrafilter type PM 10. The filter effects retention of material with a molecular weight exceeding 10 000. The ultrafiltration was run at a rate of 2.5 ml per sq.cm per hour at room temperature, using a pressure of 1,4 atm. of nitrogen.

Due to the low viscosity of the aqueous fraction of the autolysate, the ultrafiltration proceeded very effectively. Ultrafiltration was continued until the volume of the autolysate had been reduced to one tenth of the original volume. The high-molecular weight fraction after ultrafiltration contained the enzyme activity, whereas the permeate contained low-molecular weight material, mainly free amino acids and other break-down products after autolysis.

5

Example 3

Squeezed krill was obtained from whole fresh or frozen krill by pressing and centrifuging the raw material. In brief frozen krill, stored at minus 20-30 °C, was allowed to thaw at room temperature for 20 hrs and then centrifuged for 10-30 min at 1 500-3 000 xg to remove insoluble substances. The viscous liquid was collected and defined as squeezed krill. The fresh krill was processed in the same way without thawing.

1 000 ml of squeezed krill were subjected to spontaneous autolysis by storing at different temperatures (20-45 °C) and times (10-48 hrs) at different pH-values 6,8-7,0. After terminated incubation the mixtures were centrifuged at 3 500 xg for 60 min. This resulted in partitioning the material into three distinct phases for pH below 8. The middle phase represented by a clear aqueous liquid contained, as in example 2, high levels of hydrolytic enzymes degrading proteins, polysaccharides and polynucleotides. This was removed by sucking and subjected to concentration/purification by membrane filtration. The high molecular weight substances (>10 000 Daltons) were further purified by ion exchange chromatography (e.g. Q-Sepharose®, Pharmacia AB, Sweden) or hydrophobic interaction chromatography (e.g. Phenyl Sepharose® or Alkyl Sepharose®) using continuous or discontinuous salt gradients when eluting different enzymes/proteins. The enzymatic activity was collected for different enzyme groups and desalted by gel filtration or dialysis procedures. In this matter one or several bulk enzyme mixtures were obtained for further isolation and purification of individual hydrolytic enzymes.

The protein content, total proteolytic activity, trypsin-like activity and hyaluronidase activity were followed during the process, see table I-IV. In addition amylase, beta-glucuronidase, endo-(1,3)-beta-D-glucanase and carboxypeptidase activities were measured.

EP 0 393 035 B1

Table_I

Summary of autolysis (25°C; 20 h ) and partial purification of squeezed krill by membrane filtration and anion exchange chromatography on Q-Sepharose FF

| Sample | Volume | Protein | | Caseinolytic activity | | | Trypsin-like activity | | |
|---|---|---|---|---|---|---|---|---|---|
| | | | | Total units | Recovery | Purification | Total units | Recovery | Purification |
| | (ml) | (g) | (%) | | (%) | (fold) | | (%) | (fold) |
| Squeezed krill | 1000 | 40 | 100 | 1500 | 100 | 1 | 32000 | 100 | 1 |
| Autolysate | 760 | 17 | 42 | 1250 | 83 | 2 | 32000 | 100 | 2.4 |
| Protein concentrate | 95 | 6 | 15 | 1150 | 77 | 5 | 29600 | 92 | 6 |
| Enzyme pool after ion exchange chromatography | 250 | 1.5 | 4 | 900 | 60 | 16 | 21700 | 68 | 18 |

Table_II

Summary of further purification of serine proteinases from a bulk enzyme mixture isolated from autolysed squeezed krill

| Step | Volume (ml) | Protein | | Trypsin-like activity | | | Recovery (%) | Purification (fold) |
|---|---|---|---|---|---|---|---|---|
| | | (mg/ml) | Total mg | (U/ml) | Total units | Spec. act. (U/mg protein) | | |
| Bulk enzyme | 9 | 6.5 | 58.5 | 68 | 612 | 10.5 | 100 | 1 |
| Benzamidine-Sepharose 6B | 21 | 0.5 | 10.5 | 25 | 525 | 50 | 86 | 4.8 |
| Sephadex G-25 | 43 | 0.24 | 10.3 | 10 | 430 | 42 | 70 | 4 |
| Protein concentrate | 8.6 | 0.8 | 6.9 | 43 | 370 | 54 | 60 | 5.1 |

## Table III

### Protein degradation

<table>
<tr><td colspan="2" align="center">Effect of temperature</td><td colspan="2" align="center">Effect of time</td></tr>
<tr><td>Temperature<br>(°C)</td><td>Protein residues<br>(%)</td><td>Time<br>(h)</td><td>Protein residues<br>(%)</td></tr>
<tr><td>8</td><td>100<br>(no autolysis)</td><td>0</td><td>100</td></tr>
<tr><td>20</td><td>12-21</td><td>10</td><td>20-25</td></tr>
<tr><td>35</td><td>12-17</td><td>20</td><td>10-20</td></tr>
<tr><td>45</td><td>8-17</td><td>48</td><td>10-20</td></tr>
</table>

Experiments performed for 20 h.    Experiments performed at 25°C

EP 0 393 035 B1

Table IV

Summary of further purification of hyaluronidase from a bulk enzyme mixture isolated from autolysed squeezed krill

| Step | Volume (ml) | Protein | | Hyaluronidase activity | | | Recovery (%) | Purification (fold) |
|---|---|---|---|---|---|---|---|---|
| | | (mg/ml) | Total mg | (U/ml) | Total units | Spec.act. (U/mg protein) | | |
| Bulk enzyme | 77 | 5 | 385 | 140 | 10780 | 28 | 100 | 1 |
| Con A-Sepharose | 43 | 1.2 | 52 | 68 | 2924 | 57 | 27 | 2 |
| Protein concentrate YM 10 filter | 10 | 4.1 | 41 | 291 | 2910 | 71 | 27 | 2.5 |
| Superose 6 | 26 | 0.6 | 16 | 59 | 1534 | 98 | 14 | 3.5 |
| FPLC-Mono Q HR 10/10 after concentration YM 10 filter | 22 | 0.47 | 10 | 63 | 1386 | 134 | 13 | 4.8 |

Referenslista

*   Chen et al., 1978: J. Food Biochem. 2, p 349-66.
*   Chen & Gau, 1981: J. Food Biochem. 5, p 63-8.
*   Ellingsen & Mohr, 1979: Process Biochem. 14 (10), p 14.

* Ellingsen, 1982: Biochemical Studies on Antarctic Krill (Ph. D. Thesis) Department of Biochemistry, The Norwegian Institute of Technology, The University of Trondheim.
* Ellingsen & Mohr, 1987: Biochem. J. In press.
* Hellgren, Mohr & Vincent, 1986: Experentia 42, p 403-4.
* Hellgren, Mohr & Vincent, 1987: 17th World Congr. Dermatol., Berlin, p 196.
* Hellgren, Mohr & Vincent, 1985: WO-A-85/04809.
* Karlstam, 1988: EP-A-252,044 (Pharmacia AB).
* Nagayama et al., 1979: Transactions of the Tokyo University of Fisheries 3, p 153-159.
* Osnes & Mohr, 1985a: Comp. Biochem. Physiol. 82B, p 599-606.
* Osnes & Mohr, 1985b: Comp. Biochem. Physiol. 82B, p 607-19.
* Osnes & Mohr, 1986: Comp. Biochem. Physiol. 83B, p 445-458.
* Osnes, Ellingsen & Mohr, 1986: Comp. Biochem. Physiol. 83B, p 802-5.
* Rick, 1974: Methods of Enzymatic Analysis (ed.: H. Bergmeyer) 2nd edn., vol 2 pp 1013, 1023.
* Saether, 1986: Biochemistry of North Atlantic Krill. (Ph. D. Thesis) Department of Biochemistry, The Norwegian Institute of Technology, The University of Trondheim.
* Saether, Ellingsen & Mohr, 1987: Comp. Biochem. Physiol. In press.
* Van, 1982: Agric. Biol. Chem. 46, p 691-6.

## Claims

1. Method for the isolation of active enzyme(s) from an animal of the order Euphausiaceae, characterized in that the animals, or parts of the animals are induced to autolyse to the formation of distinct oil and aqueous phases, whereupon the phases are separated and the active enzyme(s) is(are) isolated from the appropriate phases by conventional methods.

2. Method according to claim 1, characterized in that the animals or part of the animals prior to the autolysis may have been unfrozen, frozen and/or homogenized, or squeezed.

3. Method according to claim 1, characterized in that autolysis is achieved by incubating the animals or part of the animals at a temperature in the range 15 to 70 °C, for a period of time ranging from 1 hour to 2 weeks.

4. Method according to anyone of claim 1-3, characterized in that autolysis is carried out at pH-values in the range pH 6 to 8.

5. Method according to anyone of claim 1-4, characterized in that the enzyme(s) is(are) isolated from the aqueous phase.

6. Method as claimed in one or more of claims 1 to 5, characterized in further processing the isolated active enzyme(s) into a preparation for medical use.

7. Method as claimed in one or more of claims 1 to 5, characterized in further processing the isolated active enzyme(s) into a preparation for technical use.

## Patentansprüche

1. Verfahren zum Isolieren eines aktiven Enzyms bzw. von aktiven Enzymen aus einem Tier der Ordnung Euphausiceae, dadurch gekennzeichnet, dass die Tiere oder Teile der Tiere dazu gebracht werden, unter Bildung von getrennten öligen und wässrigen Phasen zu autolysieren, worauf die Phasen getrennt werden und das aktive Enzym bzw. die aktiven Enzyme durch herkömmliche Methoden aus den geeigneten Phasen isoliert wird bzw. werden.

2. Verfahren nach Anspruch 1, dadurch gekennzeichnet, dass die Tiere oder Teile der Tiere vor der Autolyse aufgetaut, gefroren und/oder homogenisiert oder zerdrückt werden konnten.

3. Verfahren nach Anspruch 1, dadurch gekennzeichnet, dass die Autolyse durch Inkubation der Tiere oder Teile der Tiere bei einer Temperatur im Bereich von 15 bis 70 °C während einer Zeitdauer im Bereich von 1 Stunde bis 2 Wochen erfolgt.

4. Verfahren nach einem der Ansprüche 1 - 3, dadurch gekennzeichnet, dass die Autolyse bei pH-Werten im Bereich von pH 6 bis 8 durchgeführt wird.

5. Verfahren nach einem der Ansprüche 1 - 4, dadurch gekennzeichnet, dass das Enzym bzw. die Enzyme aus der wässrigen Phase isoliert wird bzw. werden.

6. Verfahren nach einem oder mehreren der Ansprüche 1 - 5, gekennzeichnet durch eine Weiterverarbeitung des bzw. der isolierten Enzyms bzw. Enzyme zu einem Präparat für medizinische Verwendung.

7. Verfahren nach einem oder mehreren der Ansprüche 1 - 5, gekennzeichnet durch eine Weiterverarbeitung des bzw. der isolierten Enzyms bzw. Enzyme zu einem Präparat für technische Verwendung.

**Revendications**

1. Procédé pour l'isolation d'enzyme(s) active(s) à partir d'un animal de l'ordre Euphausiacease, carctérisé en ce que les animaux ou des parties des animaux sont soumis à une autolyse jusqu'à la formation de phases huileuse et aqueuse distinctes, après quoi les phases sont séparées et la ou les enzyme(s) active(s) est (sont) isolée(s) des phases appropriées par des procédés classiques.

2. Procédé selon la revendication 1, caractérisé en que les animaux ou une partie des animaux avant l'autolyse peuvent être non congelés, congelés et/ou homogénéisés ou comprimés.

3. Procédé selon la revendication 1, caractérisé en ce que l'autolyse est obtenue par incubation des animaux ou d'une partie des animaux à une température dans la gamme de 15 à 70°C pendant une période de temps allant de 1 heure à 2 semaines.

4. Procédé selon l'une quelconque des revendications 1 à 3, caractérisé en ce que l'autolyse est mise en oeuvre à des valeurs de pH allant de pH 6 à 8.

5. Procédé selon l'une quelconque des revendications 1 à 4, caractérisé en ce que la ou les enzyme(s) est (sont) isolée(s) à partir de la phase aqueuse.

6. Procédé tel que revendiqué dans l'une ou plusieurs des revendications 1 à 5, caractérisé en ce que la ou les enzyme(s) active(s) isolée(s) sont transformées en une préparation à usage médical.

7. Procédé tel que revendiqué dans l'une ou plusieurs des revendications 1 à 5, caractérisé en ce que la ou les enzyme(s) active(s) isolée(s) sont transformées en une préparation à usage technique.